(19) Europäisches Patentamt / European Patent Office / Office européen des brevets

(11) **EP 4 223 226 B1**

(12) **EUROPEAN PATENT SPECIFICATION**

(45) Date of publication and mention
of the grant of the patent:
**08.10.2025  Bulletin 2025/41**

(21) Application number: **21875229.3**

(22) Date of filing: **15.09.2021**

(51) International Patent Classification (IPC):
*A61B 8/00* (2006.01)        *H04R 17/00* (2006.01)
*H04R 31/00* (2006.01)       *B06B 1/06* (2006.01)
*G10K 11/02* (2006.01)       *A61B 5/00* (2006.01)
*C08K 3/013* (2018.01)       *C08K 3/08* (2006.01)
*C08K 3/22* (2006.01)        *C08L 63/00* (2006.01)
*G01N 29/28* (2006.01)

(52) Cooperative Patent Classification (CPC):
**C08L 63/00; A61B 5/0095; B06B 1/067;
C08K 3/013; C08K 3/08; C08K 3/22; G01N 29/28;
G10K 11/02**

(86) International application number:
**PCT/JP2021/033982**

(87) International publication number:
**WO 2022/070927 (07.04.2022 Gazette 2022/14)**

(54) **ACOUSTIC MATCHING LAYER MATERIAL, ACOUSTIC MATCHING SHEET, COMPOSITION FOR FORMING ACOUSTIC MATCHING SHEET, ACOUSTIC WAVE PROBE, ACOUSTIC WAVE MEASUREMENT DEVICE, AND PRODUCTION METHODS FOR ACOUSTIC MATCHING LAYER MATERIAL AND ACOUSTIC WAVE PROBE**

AKUSTISCHES ANPASSUNGSSCHICHTMATERIAL, AKUSTISCHE ANPASSUNGSSCHICHT, ZUSAMMENSETZUNG ZUR BILDUNG EINER AKUSTISCHEN ANPASSUNGSFOLIE, AKUSTISCHE WELLENSONDE, AKUSTISCHE WELLENMESSVORRICHTUNG UND HERSTELLUNGSVERFAHREN FÜR AKUSTISCHES ANPASSUNGSSCHICHTMATERIAL UND AKUSTISCHE WELLENSONDE

MATÉRIAU DE COUCHE D'ADAPTATION ACOUSTIQUE, FEUILLE D'ADAPTATION ACOUSTIQUE, COMPOSITION DESTINÉE À FORMER UNE FEUILLE D'ADAPTATION ACOUSTIQUE, SONDE D'ONDE ACOUSTIQUE, DISPOSITIF DE MESURE D'ONDE ACOUSTIQUE, ET PROCÉDÉS DE PRODUCTION DE MATÉRIAU DE COUCHE D'ADAPTATION ACOUSTIQUE ET DE SONDE D'ONDE ACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2020  JP 2020166114**

(43) Date of publication of application:
**09.08.2023  Bulletin 2023/32**

(73) Proprietor: **FUJIFILM Corporation
Tokyo 106-8620 (JP)**

(72) Inventor: **HAMADA, Kazuhiro
Ashigarakami-gun, Kanagawa 258-8577 (JP)**

(74) Representative: **HGF
HGF Limited
1 City Walk
Leeds LS11 9DX (GB)**

(56) References cited:
**WO-A1-2019/088145       WO-A1-2019/088148
JP-A- 2013 192 113       JP-A- 2014 168 489
JP-A- 2017 012 436       JP-A- 2019 114 979
US-A1- 2007 205 698       US-A1- 2015 173 625
US-A1- 2020 255 652**

**Description**

**BACKGROUND OF THE INVENTION**

1. Field of the Invention

The present invention relates to an acoustic matching layer material, an acoustic matching sheet, a composition for forming an acoustic matching sheet, an acoustic wave probe, an acoustic wave measurement apparatus, and manufacturing methods of an acoustic matching layer material and an acoustic wave probe.

2. Description of the Related Art

In an acoustic wave measurement apparatus, an acoustic wave probe is used which irradiates a test object such as a living body with an acoustic wave, receives a reflected wave (echo) therefrom, and outputs a signal. The reflected wave received by this acoustic wave probe is converted into an electric signal which is displayed as an image. Therefore, by using the acoustic wave probe, it is possible to visualize and observe an inside of the test object.

An ultrasonic wave, a photoacoustic wave, or the like is appropriately selected as the acoustic wave according to the test object or measurement conditions.

For example, an ultrasound diagnostic apparatus, which is a kind of the acoustic wave measurement apparatus, transmits an ultrasonic wave to the inside of the test object, receives the ultrasonic wave reflected by tissues inside the test object, and displays the received ultrasonic wave as an image.

In addition, a photoacoustic wave measurement apparatus, which is a kind of the acoustic wave measurement apparatus, receives an acoustic wave radiated from the inside of the test object due to a photoacoustic effect, and displays the received acoustic wave as an image. The photoacoustic effect is a phenomenon in which an acoustic wave (typically, an ultrasonic wave) is generated through thermal expansion after the test object absorbs an electromagnetic wave to generate heat in a case where the test object is irradiated with an electromagnetic wave pulse of visible light, near infrared light, microwave, or the like.

Since the acoustic wave measurement apparatus transmits and receives an acoustic wave to and from the test object, the acoustic wave probe is required to match an acoustic impedance with the test object (typically, a human body). In order to satisfy this requirement, the acoustic wave probe is provided with an acoustic matching layer. This will be described by taking, as an example, a probe for an ultrasound diagnostic apparatus (also referred to as an ultrasound probe), which is a kind of the acoustic wave probe.

The ultrasound probe includes a piezoelectric element that transmits and receives an ultrasonic wave and an acoustic lens which comes into contact with a living body, in which an acoustic matching layer is disposed between the piezoelectric element and the acoustic lens. An ultrasonic wave oscillated from the piezoelectric element is incident on the living body after being transmitted through the acoustic matching layer, further being transmitted through the acoustic lens. There is usually a difference in acoustic impedance (density x acoustic velocity) between the acoustic lens and the living body. In a case where this difference is large, the ultrasonic wave is easily reflected on a surface of the living body, and an incident efficiency of the ultrasonic wave into the living body is lowered. Therefore, the acoustic lens is required to have an acoustic impedance characteristic close to that of the living body.

On the other hand, the difference in acoustic impedance between the piezoelectric element and the living body is generally large. Accordingly, the difference in acoustic impedance between the piezoelectric element and the acoustic lens is also usually large. Therefore, in a case of a laminated structure of the piezoelectric element and the acoustic lens, the ultrasonic wave emitted from the piezoelectric element is reflected on a surface of the acoustic lens, and the incident efficiency of the ultrasonic wave into the living body is lowered. In order to suppress this reflection of the ultrasonic wave, the above-described acoustic matching layer is provided between the piezoelectric element and the acoustic lens. The acoustic impedance of the acoustic matching layer takes a value between the acoustic impedance of the living body or the acoustic lens and the acoustic impedance of the piezoelectric element, which leads to improved propagation efficiency of an ultrasonic wave from the piezoelectric element to the living body. In addition, in recent years, the development of an acoustic matching layer with more efficient propagation of an ultrasonic wave has been underway by providing a gradient in acoustic impedance from the piezoelectric element side to the acoustic lens side, through a configuration of an acoustic matching layer having a multi-layer structure in which a plurality of acoustic matching sheets (sheet-like acoustic matching layer materials) are laminated.

The acoustic impedance of the acoustic matching layer can be increased to a desired level by containing an inorganic filler such as metal particles in the acoustic matching layer. For example, WO2019/088145A discloses an acoustic matching layer which contains a component derived from a binding material including a resin such as an epoxy resin and contains metal particles having a specific monodispersity. In addition, JP2014-168489A discloses an acoustic matching layer which contains a component derived from an epoxy resin and an inorganic filler such as ferrite. Patent documents US2007/205698 and US 2015/173625 refer to acoustic matching materials comprising a thermosetting resin component and inorganic filler particles of different sizes.

**SUMMARY OF THE INVENTION**

In the acoustic matching layer having a multi-layer structure, the above-described gradient of the acoustic impedance is designed such that the closer it is to the piezoelectric element, the larger the acoustic impedance of the acoustic matching sheet, and the closer it is to the acoustic lens, the smaller the acoustic impedance of the acoustic matching sheet. That is, an acoustic matching sheet having acoustic impedance close to the acoustic impedance of the piezoelectric element (usually, approximately 25 Mrayl) is required on the piezoelectric element side; and an acoustic matching sheet having acoustic impedance close to the acoustic impedance of the living body (1.4 to 1.7 Mrayl in the human body) is required on the acoustic lens side.

The acoustic impedance of the acoustic matching sheet is determined by multiplying a density and an acoustic velocity of a sheet constituent material. Therefore, in a case of trying to increase the acoustic impedance of the acoustic matching sheet used on the piezoelectric element side, it is conceivable to use a material having a high density and a high acoustic velocity. However, in a case where a filler such as a metal having a high density is contained in the acoustic matching sheet in order to increase the acoustic impedance, it has been found that, while the density of the sheet can be improved, the acoustic velocity of the sheet decreases. Therefore, in a case where an inorganic filler such as a metal having a high density is used for the acoustic matching sheet, in order to use this sheet on the piezoelectric element side, a technique for suppressing the decrease in acoustic velocity or a technique for increasing the acoustic velocity has been required.

An object of the present invention is to provide an acoustic matching layer material according to claim 1, in which, while using inorganic filler particles, it is possible to effectively increase an acoustic velocity of an acoustic matching sheet to be obtained, and it is also possible to suppress variations in acoustic characteristics in this acoustic matching sheet.

Another object of the present invention is to provide an acoustic matching sheet according to claim 8, in which, while using inorganic filler particles, an acoustic velocity is effectively increased and variations in acoustic characteristics in the sheet are suppressed, and a composition for forming an acoustic matching sheet, which is suitable for forming this acoustic matching sheet.

Another object of the present invention is to provide an acoustic wave probe according to claim 10, using the acoustic matching sheet, and an acoustic wave measurement apparatus according to claim 11, using the acoustic wave probe.

Another object of the present invention is to provide a manufacturing method of the acoustic matching layer material according to claim 13, and a manufacturing method of an acoustic wave probe using the acoustic matching layer material, according to claim 14.

As a result of studying in view of the above-described objects, the present inventors have found that, by including inorganic filler particles having a specific size in a specific proportion in the acoustic matching layer material, the acoustic velocity of the acoustic matching layer to be obtained can be effectively increased, and the variations in acoustic characteristics can be reduced. The present invention has been completed by further repeating studies on the basis of the above-described finding.

Further preferred embodiments of the invention are defined by the dependent claims.

In the present specification, the above-described "a" to "c" are values determined by a method described in Examples later.

In addition, the expression "to" in the present specification is used to mean that numerical values described before and after "to" are included as a lower limit value and an upper limit value, respectively.

With the acoustic matching layer material according to the aspect of the present invention, while using inorganic filler particles, it is possible to effectively increase an acoustic velocity of an acoustic matching sheet to be obtained, and it is also possible to suppress variations in acoustic characteristics in this acoustic matching sheet.

In addition, with the acoustic matching sheet according to the aspect of the present invention, while using inorganic filler particles, an acoustic velocity is effectively increased and variations in acoustic characteristics in the sheet are suppressed.

By curing the composition for forming an acoustic matching sheet according to the aspect of the present invention, the above-described acoustic matching sheet can be obtained.

In addition, the acoustic wave probe and the acoustic wave measurement apparatus according to the aspects of the present invention include the acoustic matching sheet with the above-described excellent characteristics.

In addition, with the manufacturing method of an acoustic matching layer material according to the aspect of the present invention, the above-described material for an acoustic matching layer can be obtained. With the manufacturing method of the acoustic wave probe according to the aspect of the present invention, an acoustic wave probe using the above-described material for an acoustic matching layer can be obtained.

## BRIEF DESCRIPTION OF THE DRAWINGS

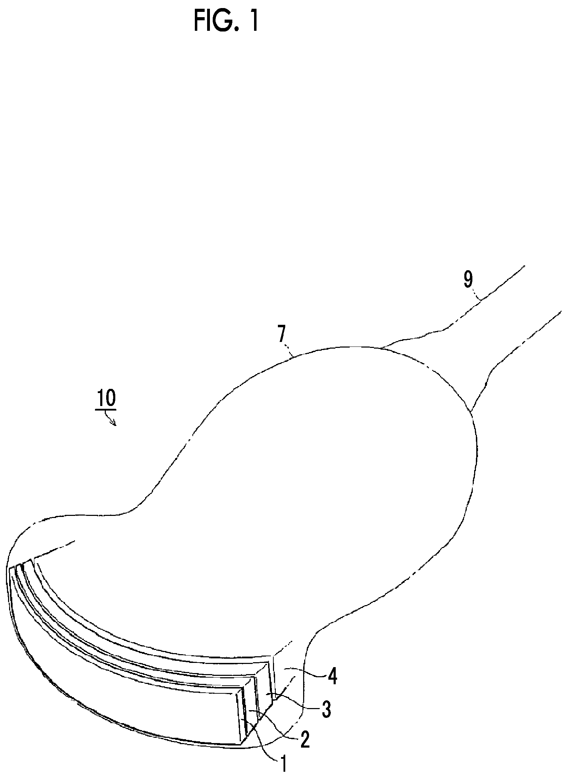

Fig. 1 is a perspective view of an example of a convex type ultrasound probe which is an aspect of an acoustic wave probe.

## DESCRIPTION OF THE PREFERRED EMBODIMENTS

<Acoustic matching layer material>

An acoustic matching layer material according to an embodiment of the present invention (hereinafter, also simply referred to as a "layer material according to the embodiment of the present invention") is a material obtained by curing a composition for forming an acoustic matching layer, which will be described later, and may be a sheet material used as an acoustic matching layer as it is, or may be a material in a shape prior to processing into the shape of the acoustic matching layer (for example, an aspect of a sheet which is thicker than a thickness of the acoustic matching layer, in which this thick sheet material is sliced and used as the acoustic matching layer).

The layer material according to the embodiment of the present invention includes a thermosetting resin component and inorganic filler particles, in which in a cross-sectional observation of the acoustic matching layer material, a cross-sectional area of the inorganic filler particles satisfies Expression (1).

$$\text{Expression (1)} \quad 100 \times b/c \geq 25$$

b: in a case where a number-average value of cross-sectional areas of the inorganic filler particles is denoted as a, a total cross-sectional area of particles having a cross-sectional area 7 times or more of a in the inorganic filler particles is denoted as b.

c: total cross-sectional area of the inorganic filler particles is denoted as c.

That is, the proportion of the b to the b is 25% or more.

The above-described "a" to "c" are cross-sectional areas of the inorganic filler particles in the cross-sectional observation of the acoustic matching layer material by an ion milling method using a cross-section polisher, which is as described in Examples later.

With the acoustic matching layer material according to the aspect of the present invention, while using inorganic filler particles, it is possible to effectively increase an acoustic velocity of an acoustic matching sheet to be obtained, and it is also possible to suppress variations in acoustic characteristics in this acoustic matching sheet. The reason for this is not clear, but it is presumed as follows.

The layer material according to the embodiment of the present invention includes the thermosetting resin component as a matrix, and includes the inorganic filler particles having a specific spread in size distribution in the matrix. That is, in the matrix, a certain proportion of the inorganic filler particles are in contact (connected) with each other and are dispersed in a large particle size, and a transmission speed of acoustic wave between the inorganic filler particles or between the inorganic filler particles and the thermosetting resin component is effectively increased. On the other hand, a certain amount of small-sized inorganic filler particles which are not in contact (not connected) with each other are also dispersed, and it is considered that the small-sized inorganic filler particles contribute to uniformity of the acoustic matching layer material as a whole, which effectively suppresses variations in acoustic characteristics.

Expression (1) is preferably Expression (2), more preferably Expression (3), and still more preferably Expression (4).

$$\text{Expression (2)} \quad 100 \times b/c \geq 30$$

$$\text{Expression (3)} \quad 80 \geq 100 \times b/c \geq 30$$

$$\text{Expression (4)} \quad 80 \geq 100 \times b/c \geq 50$$

The shape of the layer material according to the embodiment of the present invention is not particularly limited, and examples thereof include a sheet shape, a columnar shape, and a prismatic shape, and a sheet shape is preferable.

Hereinafter, the thermosetting resin component may be referred to as a "binding material". In this case, in a case where the layer material according to the embodiment of the present invention includes a curing agent component (component derived from a curing agent), the thermosetting resin component and the curing agent component are collectively referred to as the "binding material".

The "thermosetting resin" is a resin having a property of being cured by heat, and the "thermosetting resin component" is a component in a state in which the thermosetting resin is cured by heat (for example, a state in which the thermosetting resin is cured by reacting with the curing agent by heat).

(Thermosetting resin)

The thermosetting resin which derives the thermosetting resin component contained in the layer material according to the embodiment of the present invention is not particularly limited. Examples thereof include an epoxy resin, a phenol resin, a melamine resin, a urea resin, an unsaturated polyester resin, an alkyd resin, a thermosetting polyurethane resin, and a thermosetting polyimide resin.

Among these, from the viewpoint of further suppressing the variations in acoustic characteristics of the acoustic matching layer material to be obtained, the thermosetting resin preferably includes an epoxy resin. In this case, it is preferable to combine the epoxy resin with a curing agent. That is, it is preferable to apply the epoxy resin as the thermosetting resin and to further use a curing agent in combination. In this case, the layer material according to the embodiment of the present invention includes a binding material (thermosetting resin component) including an epoxy resin component and a curing agent component.

In a case where the epoxy resin is used as the thermosetting resin, it is preferable that the epoxy resin includes one or two or more of a bisphenol A type epoxy resin, a bisphenol F type epoxy resin, and a phenol novolac type epoxy resin.

The bisphenol A type epoxy resin used in the present invention is not particularly limited, and any bisphenol A type epoxy resin commonly used as a main agent of an epoxy-based adhesive can be widely used. Preferred specific examples thereof include bisphenol A diglycidyl ethers (jER825, jER828, and jER834 (all trade names), manufactured by Mitsubishi Chemical Corporation) and bisphenol A propoxylate diglycidyl ethers (manufactured by Sigma-Aldrich Co. LLC).

The bisphenol F type epoxy resin used in the present invention is not particularly limited, and any bisphenol F type epoxy resin commonly used as a main agent of an epoxy-based adhesive can be widely used. Preferred specific examples thereof include bisphenol F diglycidyl ether (trade name: EPICLON 830, manufactured by DIC Corporation) and 4,4'-methylenebis(N,N-diglycidylaniline).

The phenol novolac type epoxy resin used in the present invention is not particularly limited, and any phenol novolac type epoxy resin commonly used as a main agent of an epoxy-based adhesive can be widely used. Such a phenol novolac type epoxy resin is commercially available, for example, by the product number 406775 from Sigma-Aldrich Co. LLC.

As the curing agent, one known as a curing agent for an epoxy resin can be used without particular limitation. Examples thereof include an aliphatic amine, an aromatic amine, polyamide amine, polyether amine, dicyandiamide, a dihydrazide compound, an acid anhydride, and a phenolic resin. Among these, from the viewpoint of slowing a curing rate and improving a pot life of the mixture, it is preferable to use at least one of a primary amine or a secondary amine. A primary amine is more preferable.

(Inorganic filler particles)

The layer material according to the embodiment of the present invention contains inorganic filler particles. By adjusting a content of the inorganic filler particles in the layer material, a density of the layer material can be adjusted, and an acoustic impedance of the layer material can be adjusted to a desired level.

In addition, from the viewpoint of increasing the acoustic impedance of the layer material by increasing the density of the layer material, a density of the inorganic filler particles (25°C, g/cm$^3$) is preferably 4.0 or more, more preferably 7.5 or more, and still more preferably 15 or more. In addition, the density of the inorganic filler particles is preferably 21.5 or less.

The inorganic filler particles are not particularly limited, and preferably include, for example, metal particles. A content of the metal particles in the inorganic filler particles is not particularly limited, but is preferably 80% by mass or more, more preferably 90% by mass or more, and still more preferably 95% by mass or more, and may be 100% by mass.

The inorganic filler particles may be surface-treated. The surface treatment can be performed, for example, with reference to WO2019/088148A.

In a case where the inorganic filler particles include the metal particles, metal atoms constituting the metal particles may be contained alone, or may be contained in a state of a carbide, a nitride, an oxide, or a boronized metal. In addition, an alloy may be formed. Examples of the type of the alloy include high tension steel (Fe-C), chromium molybdenum steel (Fe-Cr-Mo), manganese molybdenum steel (Fe-Mn-Mo), stainless steel (Fe-Ni-Cr), 42 alloy, invar (Fe-Ni), permendur (Fe-Co), silicon steel (Fe-Si), gunmetal, tombac (Cu-Zn), nickel silver (Cu-Zn-Ni), bronze (Cu-Sn), cupronickel (Cu-Ni), red copper (Cu-Au), constantan (Cu-Ni), Deralumin (Al-Cu), HASTELLOY (Ni-Mo-Cr-Fe), Monel (Ni-Cu), Inconel (Ni-Cr-Fe), Nichrome (Ni-Cr), Ferromanganese (Mn-Fe), cemented carbide (WC/Co), and tungsten carbide (W-C).

The above-described metal atoms preferably include at least one metal atom of periodic table Groups 4 to 12, and more preferably include at least one metal atom of periodic table Groups 4 to 9. The above-described metal atoms more preferably include at least one kind of Ti, Ag, Pt, Fe, Co, Zr, Mo, and W, and more preferably include at least one kind of Fe, Co, Zr, Mo, and W. A content of the metal atom of periodic table Groups 4 to 12 in the metal atoms constituting the metal particles is not particularly limited, and the total thereof is preferably 80% by mass or more, more preferably 90% by mass or more, and still more preferably 95% by mass or more, and may be 100% by mass.

In the acoustic matching layer material according to the embodiment of the present invention, the number-average value a of the cross-sectional areas of the inorganic filler particles may be, for example, $1 \times 10^{-5}$ to $2 \times 10^4$ $\mu m^2$, $3 \times 10^{-3}$ to $2 \times 10^3$ $\mu m^2$, or $7 \times 10^{-2}$ to $2 \times 10^2$ $\mu M^2$.

In the layer material according to the embodiment of the present invention, respective contents of the inorganic filler particles and the binding material are appropriately adjusted in accordance with a target acoustic impedance and the like. For example, in a case where the acoustic matching layer is formed into a multi-layer, the content of the metal particles in the layer material used for the acoustic matching layer on the piezoelectric element side can be relatively high, and the content of the metal particles in the layer material used for the acoustic matching layer on the acoustic lens side can be relatively low. As a result, the acoustic impedance can be slanted from the piezoelectric element side toward the acoustic lens side, and the propagation of the acoustic wave can be more efficient.

In the layer material according to the embodiment of the present invention, the content of the inorganic filler particles is appropriately adjusted as described above, and is usually 50% to 98% by mass, preferably 60% to 96% by mass and more preferably 65% to 96% by mass.

In addition, the content of the binding material in the layer material is usually 2% to 50% by mass, preferably 4% to 40% by mass more preferably 4% to 35% by mass.

The layer material according to the embodiment of the present invention may be composed of the binding material and the inorganic filler particles. In addition, a component other than the binding material and the inorganic filler particles may be contained as long as the effects of the present invention are not impaired. Examples of the component other than the binding material and the inorganic filler particles include those derived from a component which does not correspond to any of the thermosetting resin, the inorganic filler particles, or the curing agent, which is contained in the composition for forming an acoustic matching layer described later.

In the layer material according to the embodiment of the present invention, the total content of the binding material and the inorganic filler particles is preferably 80% by mass or more and more preferably 90% by mass or more, and may be 100% by mass.

<Acoustic matching sheet (acoustic matching layer)>

An acoustic matching sheet can be obtained by cutting, dicing, or the like the layer material according to the embodiment of the present invention into a desired thickness or shape, as necessary. In addition, the acoustic matching sheet can be further processed into a desired shape by a conventional method.

For example, the composition according to the embodiment of the present invention, which will be described later, is shaped into a desired sheet in a low temperature region where a curing reaction does not occur or in a low temperature region where a curing rate is sufficiently slow. Next, the material is subjected to isotropically pressing, and heated and cured as necessary to form a crosslinking structure in a molded product, and an acoustic matching sheet or a precursor sheet thereof is obtained by cutting, dicing, or the like into a desired thickness or shape, as necessary. That is, the acoustic matching sheet to be formed is preferably a cured substance (that is, a sheet formed from the acoustic matching layer material) obtained by curing the composition according to the embodiment of the present invention to form a three-dimensional network structure. This acoustic matching sheet is used as an acoustic matching layer of an acoustic wave probe. The configuration of the acoustic wave probe including the acoustic matching layer will be described later.

<Composition for forming acoustic matching layer>

The composition for forming an acoustic matching layer according to the embodiment of the present invention (composition used for the acoustic matching layer material according to the embodiment of the present invention (composition for forming the acoustic matching layer material according to the embodiment of the present invention); also referred to as a "composition for forming an acoustic matching sheet" and may be simply referred to as a "composition according to the embodiment of the present invention") contains a thermosetting resin and inorganic filler particles.

In addition, the composition according to the embodiment of the present invention may contain the above-described curing agent, and can appropriately contain a component that is not the thermosetting resin, the inorganic filler particles, and the curing agent, for example, at least one of a curing retarder, a solvent, a dispersing agent, a pigment, a dye, an antistatic agent, an antioxidant, a flame retardant, a thermal conductivity improver, and the like.

In a case where the composition according to the embodiment of the present invention contains an epoxy resin as the thermosetting resin and a curing agent of the epoxy resin, even under mild conditions, the curing reaction of the epoxy resin may progress over time in the composition. Therefore, properties of the composition may change with time and may not be stable. However, for example, by storing the above-described composition at a temperature of -10°C or lower, it is possible to obtain a composition in a state in which each component is stably maintained without causing the curing reaction or by sufficiently suppressing the curing reaction.

In addition, in a case where the epoxy resin is used as the thermosetting resin, it is also preferable to be an aspect in which a resin composition containing the epoxy resin and the inorganic filler particles is used as a main agent, and this main agent and the curing agent are separated in different forms. In forming the acoustic matching layer, the composition according to the embodiment of the present invention is prepared by mixing the main agent and the curing agent, a layer is formed of this composition, whereby an acoustic matching layer or a sheet-like acoustic matching layer material (acoustic matching sheet) can be formed.

In the composition according to the embodiment of the present invention, a mass ratio of the epoxy resin to the curing agent may be appropriately adjusted according to the type of the curing agent used, and the like. For epoxy resin, the epoxy resin/curing agent can be set to 99/1 to 20/80, preferably 90/10 to 40/60.

In addition, in the above-described aspect in which the main agent and the curing agent are separated in different forms, in a case of preparing the composition according to the embodiment of the present invention by mixing the main agent and the curing agent during layer formation, it is preferable to prepare the composition according to the embodiment of the present invention by mixing the main agent and the curing agent such that the mass ratio of the epoxy resin to the curing agent is epoxy resin/curing agent = 99/1 to 20/80, and it is more preferable to prepare the composition according to the embodiment of the present invention by mixing the main agent and the curing agent such that the mass ratio of the epoxy resin to the curing agent is epoxy resin/curing agent = 90/10 to 40/60.

<Preparation of composition for forming acoustic matching layer>

The composition for forming an acoustic matching layer according to the embodiment of the present invention can be obtained, for example, by mixing and homogenizing each component constituting the composition for forming an acoustic matching layer. The mixing method is not particularly limited as long as each component can be mixed substantially homogeneously. For example, a desired homogeneous mixing can be achieved by kneading using a rotation and revolution stirrer.

In addition, in a case of an aspect in which a main agent consisting of a resin composition containing the thermosetting resin and the inorganic filler particles and the curing agent of the thermosetting resin are separated in different forms, the main agent can be obtained by mixing the thermosetting resin and the inorganic filler particles. In producing the acoustic matching layer material, the composition for an acoustic matching layer material according to the embodiment of the present invention is obtained by mixing the main agent and the curing agent. The acoustic matching layer material or a precursor thereof can be prepared by curing this composition while molding the composition.

<Manufacturing method of material for acoustic matching layer>

The manufacturing method of an acoustic matching layer material according to an embodiment of the present invention is a manufacturing method of an acoustic matching layer material including a thermosetting resin component and inorganic filler particles. In a cross-sectional observation of the acoustic matching layer material, a cross-sectional area of the inorganic filler particles satisfies Expression (1). The manufacturing method preferably includes isotropically pressing a composition for forming an acoustic matching layer, which contains the thermosetting resin and the inorganic filler particles, and contains the curing agent as necessary.

$$\text{Expression (1)} \quad 100 \times b/c \geq 25$$

b: in a case where a number-average value of cross-sectional areas of the inorganic filler particles is denoted as a, a total cross-sectional area of particles having a cross-sectional area 7 times or more of a in the inorganic filler particles is denoted as b.

c: total cross-sectional area of the inorganic filler particles is denoted as c.

The isotropically pressing may be performed at cold (0°C to 30°C) or hot (40°C to 100°C). The isotropically pressing can be performed as follows, for example, by placing the composition for an acoustic matching layer material in a mold.

The isotropically pressing at cold (cold isotropically pressing) can be performed under a vacuum condition of 25 to 350 MPa for 5 to 30 minutes.

The composition for an acoustic matching layer material can be subjected to a curing reaction (crosslinking reaction) by, after the cold isotropically pressing, heating the composition at 60°C to 100°C for 80 to 200 minutes, as necessary.

The isotropically pressing at hot (hot isotropically pressing) can be performed, for example, under a vacuum condition of 25 to 350 MPa for 5 to 200 minutes.

The composition may be preliminarily pressed before performing the hot isotropically pressing. This preliminary pressing can be performed, for example, under the above-described conditions of the cold isotropically pressing.

From the viewpoint of the viscosity of the composition for an acoustic matching layer material and the variations in acoustic wave characteristics of the acoustic matching layer material, a particle size of the inorganic filler particles used in the manufacturing method of an acoustic matching layer material according to the embodiment of the present invention is preferably 0.01 to 100 $\mu$m, more preferably 0.1 to 50 $\mu$m, still more preferably 0.2 to 30 $\mu$m, even more preferably 0.5 to 20 $\mu$m, and even still more preferably 1 to 10 $\mu$m. Here, the "particle size" of the inorganic filler particles means an average primary particle diameter. In a case where the inorganic filler particles are surface-treated, it is preferable that the average primary particle diameter of the surface-treated inorganic filler particles is within the above-described range.

Here, the average primary particle diameter means a volume-based median diameter. The volume-based median diameter is determined as follows.

The inorganic filler particles are added to methanol in an amount of 0.5% by mass and subjected to ultrasonic wave for 10 minutes to disperse the metal particles. A particle size distribution of the metal particles treated as described above is measured with a laser diffraction scattering-type particle size distribution analyzer (manufactured by HORIBA, Ltd., trade name: LA950V2), thereby determining the volume-based median diameter. The median diameter corresponds to a cumulative 50% in a case where the particle size distribution is represented as a cumulative distribution.

<Acoustic wave probe>

An acoustic wave probe according to an embodiment of the present invention includes the acoustic matching sheet according to the embodiment of the present invention as at least one layer of an acoustic matching layer.

An example of the configuration of the acoustic wave probe according to the embodiment of the present invention is shown in Fig. 1. The acoustic wave probe shown in Fig. 1 is an ultrasound probe in an ultrasound diagnostic apparatus. The ultrasound probe is a probe which particularly uses an ultrasonic wave as an acoustic wave in an acoustic wave probe. Therefore, a basic structure of the ultrasound probe can be applied to the acoustic wave probe as it is.

<Ultrasound probe>

An ultrasound probe 10 is a main component of the ultrasound diagnostic apparatus and has a function of generating an ultrasonic wave and transmitting and receiving an ultrasonic beam. As shown in Fig. 1, a configuration of the ultrasound probe 10 is provided in the order of an acoustic lens 1, an acoustic matching layer 2, a piezoelectric element layer 3, and a backing material 4 from a distal end portion (surface coming into contact with a living body which is a test object). In recent years, an ultrasound probe having a laminated structure in which an ultrasonic transducer (piezoelectric element) for transmission and an ultrasonic transducer (piezoelectric element) for reception are formed of materials different from each other has been proposed in order to receive high-order harmonics.

(Piezoelectric element layer)

The piezoelectric element layer 3 is a portion which generates an ultrasonic wave and in which an electrode is attached to both sides of a piezoelectric element. In a case where voltage is applied to the electrode, the piezoelectric element layer 3 generates an ultrasonic wave through repeated contraction and expansion of the piezoelectric element and through vibration.

A so-called ceramics inorganic piezoelectric body obtained by a polarization treatment of quartz crystals, single crystals such as $LiNbO_3$, $LiTaO_3$, and $KNbO_3$, thin films of ZnO and AlN, $Pb(Zr,Ti)O_3$-based sintered body, and the like is widely used as the material constituting a piezoelectric element. In general, piezoelectric ceramics such as lead zirconate titanate (PZT) with good conversion efficiency are used.

In addition, sensitivity having a wider band width is required for a piezoelectric element detecting a reception wave on a high frequency side. For this reason, an organic piezoelectric body has been used in which an organic polymer material such as polyvinylidene fluoride (PVDF) is used as the piezoelectric element being suitable for a high frequency or a wide band.

Furthermore, cMUT using micro electro mechanical systems (MEMS) technology in which an array structure, which shows excellent short pulse characteristics, excellent wideband characteristics, and excellent mass productivity and has less characteristic variations, is obtained is disclosed in JP2011-071842A or the like.

In the present invention, it is possible to preferably use any piezoelectric element material.

(Backing material)

The backing material 4 is provided on a rear surface of the piezoelectric element layer 3 and contributes to the improvement in distance resolution in an ultrasound diagnostic image by shortening the pulse width of an ultrasonic wave through the suppression of excess vibration.

(Acoustic matching layer)

The acoustic matching layer 2 is provided in order to reduce the difference in acoustic impedance between the piezoelectric element layer 3 and a test object and to efficiently transmit and receive an ultrasonic wave.

(Acoustic lens)

The acoustic lens 1 is provided to focus an ultrasonic wave in a slice direction by utilizing refraction to improve the resolution. In addition, it is necessary for the acoustic lens 1 to achieve matching of an ultrasonic wave with the acoustic impedance (1.4 to 1.7 Mrayl in a case of a human body) of a living body which is a test object after being closely attached to the living body and to reduce the amount of ultrasonic attenuation of the acoustic lens 1 itself.

That is, by using, as the material of the acoustic lens 1, a material in which the acoustic velocity is sufficiently lower than the acoustic velocity of the human body, the attenuation of ultrasonic wave is small, and the acoustic impedance is close to the value of the skin of the human body, sensitivity of transmission and reception of the ultrasonic wave is increased.

The operation of the ultrasound probe 10 having such a configuration will be described. The piezoelectric element layer 3 is resonated after applying a voltage to the electrodes provided on both sides of the piezoelectric element, and an ultrasonic signal is transmitted to a test object from the acoustic lens. During reception of the ultrasonic signal, the piezoelectric element layer 3 is vibrated using the signal (echo signal) reflected from the test object and this vibration is electrically converted into a signal to obtain an image.

<Manufacturing method of acoustic wave probe>

The acoustic wave probe according to the embodiment of the present invention can be produced by a conventional method, except that the material for an acoustic matching layer according to the present invention is used. That is, the manufacturing method of an acoustic wave probe according to the embodiment of the present invention includes forming an acoustic matching layer on a piezoelectric element using the acoustic matching sheet formed from the material for an acoustic matching layer according to the embodiment of the present invention. The piezoelectric element can be provided on the backing material by a conventional method.

In addition, an acoustic lens is formed on the acoustic matching layer by a conventional method using a material for forming an acoustic lens.

<Acoustic wave measurement apparatus>

An acoustic wave measurement apparatus according to an embodiment of the present invention includes the acoustic wave probe according to the embodiment of the present invention. The acoustic wave measurement apparatus has a function of displaying the signal intensity of a signal received by the acoustic wave probe and imaging the signal.

It is also preferable that the acoustic wave measurement apparatus according to the embodiment of the present invention is an ultrasonic measurement apparatus using an ultrasound probe.

Examples

The present invention will be described in more detail based on Examples in which an ultrasonic wave is used as an acoustic wave. The present invention is not limited to Examples except as specified in the present invention.

In the following, the blending amount of the component means a blending amount of the component itself. That is, in a case where the raw material contains a solvent, the blending amount is an amount excluding the solvent. The acoustic wave in the present invention is not limited to the ultrasonic wave, and any acoustic wave of an audible frequency may be used as long as an appropriate frequency is selected in accordance with a test object, measurement conditions, and the like.

<1> Preparation of composition for forming acoustic matching layer

Compositions for forming an acoustic matching layer shown in Table 1 below (Tables 1-1 to 1-8 are collectively referred to as Table 1) were prepared as described below.

(1) Preparation of composition for forming acoustic matching layer used in Example 1

23.9 parts by mass of an epoxy resin (bisphenol A diglycidyl ether ("jER825" (trade name) manufactured by Mitsubishi Chemical Corporation, epoxy equivalent: 170)), 6.1 parts by mass of isophorone diamine, and 70 parts by mass of iron particles were added to a container having a cylindrical inner space having a diameter of 40 mm so that a thickness after mixing was 3 mm, and mixed with a rotation and revolution device (trade name: ARV-310, manufactured by THINKY CORPORATION) to prepare a composition for forming an acoustic matching layer used in Example 1.

(2) Preparation of compositions for forming acoustic matching layer used in Examples 2 to 72 and Comparative Examples 1 to 17

Compositions for forming an acoustic matching layer used in Examples 2 to 72 and Comparative Examples 1 to 17 were prepared in the same manner as in the composition for forming an acoustic matching layer used in Example 1, except that the composition was changed as the composition shown in Table 1 below. In a case of preparation using a raw material containing a solvent, the composition for forming an acoustic matching layer was obtained by volatilizing the solvent after mixing.

<2> Production of acoustic matching sheet (sheet-like acoustic matching layer material)

(1) Production of acoustic matching sheet by cold isotropically pressing

With regard to Examples and Comparative Examples in which "Cold" is described in "Pressurization form" in Table 1 below, an acoustic matching sheet was produced as follows.
The composition for forming an acoustic matching layer was placed in a square mold (stainless steel (SUS) frame with top and bottom openings, 40 mm square, thickness: 1 mm), the mold was placed in an aluminum laminate bag, the air inside was removed by a vacuum packing machine, and the bag was sealed. The sealed bag was set in a cold isotropic press device (CL10-55-40 (trade name), manufactured by Nikkiso Co., Ltd.) and pressurized at 300 MPa for 30 minutes at 25°C. After the pressurization, the mold including the composition for forming an acoustic matching layer was taken out from the laminate bag and heated at 100°C for 180 minutes for curing, thereby obtaining an acoustic matching sheet (40 mm square, thickness: 1 mm).

(2) Production of acoustic matching sheet by hot isotropically pressing

With regard to Examples and Comparative Examples in which "Hot" is described in "Pressurization form" in Table 1 below, an acoustic matching sheet was produced as follows.
The composition for forming an acoustic matching layer was placed in a square mold (made of SUS, 40 mm square, thickness: 1 mm), the mold was placed in an aluminum laminate bag, the air inside was removed by a vacuum packing machine, and the bag was sealed. The sealed bag was set in a hot isotropic press device (MARUGOTO EKISU 500-mL type (trade name), manufactured by Toyo Koatsu Inc.) and pressurized at 50 MPa for 30 minutes at 25°C. In a pressurized state, the composition was heated at 60°C for 180 minutes for curing, thereby obtaining an acoustic matching sheet (40 mm square, thickness: 1 mm).

(3) Production of acoustic matching sheet by vacuum electric heating pressing

With regard to Comparative Examples in which "Vacuum electric heating" is described in "Pressurization form" in Table 1 below, an acoustic matching sheet was produced as follows.
The composition for forming an acoustic matching layer was placed in a square mold (made of SUS, 40 mm square, thickness: 1 mm), and Teflon (registered trademark, Dupont) sheets (thickness: 1 mm) were placed above and below the mold. The composition for forming an acoustic matching layer, which had been sandwiched between the Teflon sheets, was set in a vacuum electric heating press device 11FD (trade name, manufactured by Imoto machinery Co.,

LTD.), and after pressing for 240 minutes at 25°C under a vacuum of 9 MPa, the composition was cured by heating at 60°C for 180 minutes while under pressure, thereby obtaining an acoustic matching sheet (40 mm square, thickness: 1 mm).

(4) Production of acoustic matching sheet without pressurization step

With regard to Examples and Comparative Examples in which "-" is described in "Pressurization form" in Table 1 below, an acoustic matching sheet was produced as follows.

A raw material of the composition for forming an acoustic matching layer was added to a cylindrical container having an inner space of 40 mm in diameter so that a thickness after mixing was 1 mm, and mixed using a rotation and revolution device (trade name: ARV-310, manufactured by THINKY CORPORATION). The composition was heated at 60°C for 24 hours and then at 150°C for 60 minutes while placed in the container, thereby obtaining an acoustic matching sheet (diameter: 40 mm, thickness: 1 mm).

<3> Production of reference acoustic matching sheet

With regard to Examples and Comparative Examples in which the acoustic matching sheets were produced in (1) to (3) of "<2> Production of acoustic matching sheet" described above, using each corresponding composition for forming an acoustic matching layer, an acoustic matching sheet was produced in the same manner as in (4) of "<2> Production of acoustic matching sheet" described above, and used as a reference acoustic matching sheet in [Test Example 2] below.

[Cross section extraction and cross section analysis]

(1) By an ion milling method using a cross-section polisher (trade name: SM-09010, manufactured by JEOL Ltd.), the acoustic matching sheet produced above was cross-sectioned in a thickness direction, and three cross sections for analysis of the acoustic matching sheet were randomly formed. The cross sections were extracted by irradiating with an argon ion beam at an acceleration voltage of 5 kV for 14 hours.
(2) After carbon vapor deposition was applied to each cross section for analysis, an image (5120 × 3840 pixels) was obtained from these cross sections by a scanning electron microscope (trade name: SU8030, manufactured by Hitachi High-Tech Corporation.).
(3) For each image, using image analysis software Image J (version 1.47) (manufactured by National Institutes of Health), the image was binarized to distinguish between the binding material (thermosetting resin component and curing agent component) and the inorganic filler particles. In the analysis of each image by binarization, the magnification and threshold value of the scanning electron microscope (trade name: SU8030, manufactured by Hitachi High-Tech Corporation.) were set so that the number of counted inorganic filler particles was 1000 or more and 1200 or less. One that the particles were in contact (connected) with each other to form one mass of particles was counted as one particle. That is, as for the connected particles, the entire group of particles which were connected and integrated was counted as one particle.
(4) The number-average value of cross-sectional areas (unit: $\mu m^2$) of the particles counted above (sum of cross-sectional areas of all particles in three analysis images/number of particles) was denoted as "a", and the total cross-sectional area of all particles in the three analysis images was denoted as c.

For each image, particles having a cross-sectional area 7 times or more of the a (the particles were usually connected particles) are extracted, and the total cross-sectional area (unit: $\mu m^2$) of all particles having a cross-sectional area 7 times or more of the a was denoted as "b".

A proportion (%, 100 × b/c) of the total cross-sectional area "b" of each particle having a cross-sectional area 7 times or more of the a to the total cross-sectional area "c" of all particles was calculated.

"a" of Examples 1 to 72 was 0.07 to 200 $\mu m^2$, and "a" of Comparative Examples 1 to 17 was 0.07 to 200 $\mu m^2$.

[Test Example 1] Measurement of acoustic velocity

The ultrasonic velocity was measured at 25°C using a sing-around acoustic velocity measurement apparatus (manufactured by Ultrasonic Engineering Co., Ltd., trade name: "UVM-2 model") according to JIS Z2353 (2003). With respect to the acoustic matching sheet obtained above, having a diameter of 40 mm and a thickness of 1 mm in plan view, or having a 40 mm square and a thickness of 1 mm, three circular regions having a diameter of 1.5 cm were randomly selected, and the entire inside of these three circular regions (small probe size of a single channel) was measured. An arithmetic mean value of acoustic velocities in the above-described three circular regions was calculated. A proportion (%) of the change in acoustic velocity, obtained from the following expression, was applied to the following evaluation standard. An evaluation of A to D is acceptable in the present test. The results are shown in Table 1.

Proportion of change in acoustic velocity (%) = 100 × (Arithmetic mean value of acoustic velocity of acoustic matching sheet)/(Arithmetic mean value of acoustic velocity of reference acoustic matching sheet)

-Evaluation standard-

A: 110% or more
B: 108% or more and less than 110%
C: 105% or more and less than 108%
D: 102% or more and less than 105%
E: 101% or more and less than 102%
F: less than 100% or 101% or less

Since the acoustic matching sheets of Comparative Examples 1, 9, 10, 11, 13, 15, and 17 were not pressurized during the production, the proportion of change in acoustic velocity was not calculated.

[Test Example 2] Variations in acoustic impedance (AI)

A 9 mm × 9 mm test piece was cut out from each acoustic velocity measurement target (circle with a diameter of 1.5 cm) of Test Example 1 described above. The density of the test piece at 25°C was measured using an electronic hydrometer (manufactured by Alfa Mirage Co., Ltd., trade name: "SD-200L") in accordance with the density measurement method of Method A (underwater substitution method) described in JIS K7112 (1999). For the acoustic matching sheet of each of Examples and Comparative Examples, the acoustic impedance (density x acoustic velocity) was calculated for each of the three circular regions, the standard deviation of the three acoustic impedances (Mrayl) was determined, and the variations in acoustic characteristics were evaluated by applying them to the following evaluation standard. An evaluation of A or B is acceptable in the present test. The results are shown in Table 1.

-Evaluation standard-

A: less than 0.3 Mrayl
B: 0.3 Mrayl or more and less than 0.5 Mrayl
E: 0.5 Mrayl or more

[Table 1]

| Table 1-1 | | | C1 | C2 | C3 | C4 | C5 | E1 | E2 | E3 | C6 | E4 | E5 | E6 | E7 |
|---|---|---|---|---|---|---|---|---|---|---|---|---|---|---|---|
| Resin | Type | | A-1 | A-1 | A-1 | A-1 | A-1 | A-1 | A-1 | A-1 | A-1 | A-1 | A-1 | A-1 | A-1 |
| | Content [part by mass] | | 23.9 | 23.9 | 23.9 | 23.9 | 23.9 | 23.9 | 23.9 | 31.9 | 31.9 | 16 | 16 | 8 | 8 |
| Curing agent | Type | | B-1 | B-1 | B-1 | B-1 | B-1 | B-1 | B-1 | B-1 | B-1 | B-1 | B-1 | B-1 | B-1 |
| | Content [part by mass] | | 6.1 | 6.1 | 6.1 | 6.1 | 6.1 | 6.1 | 6.1 | 8.1 | 8.1 | 4 | 4 | 2 | 2 |

(continued)

Table 1-1

|  |  | C1 | C2 | C3 | C4 | C5 | E1 | E2 | E3 | C6 | E4 | E5 | E6 | E7 |
|---|---|---|---|---|---|---|---|---|---|---|---|---|---|---|
| Particle | Type | Fe | Fe | Fe | Fe | Fe | Fe | Fe | Fe | Fe | Fe | Fe | Fe | Fe |
|  | Content [part by mass] | 70 | 70 | 70 | 70 | 70 | 70 | 70 | 60 | 60 | 80 | 80 | 90 | 90 |
| 100 × b/c [%] | | 11 | 12 | 13 | 19 | 22 | 27 | 33 | 25 | 20 | 43 | 48 | 62 | 68 |
| Applied pressure [MPa] | | 0 | 10 | 25 | 50 | 100 | 200 | 300 | 300 | 200 | 200 | 300 | 200 | 300 |
| Pressurization form | | - | Cold | Cold | Cold | Cold | Cold | Cold | Cold | Cold | Cold | Cold | Cold | Cold |
| Acoustic velocity | | - | E | E | E | E | D | B | D | E | B | B | A | A |
| Variations in AI | | C | B | B | B | A | B | A | B | B | A | A | A | A |

Table 1-2

|  |  | E8 | E9 | E10 | E11 | E12 | E13 | E14 | E15 | E16 | E17 | E18 | E19 | E20 |
|---|---|---|---|---|---|---|---|---|---|---|---|---|---|---|
| Resin | Type | A-2 | A-2 | A-3 | A-3 | A-4 | A-4 | A-1 | A-1 | A-1 | A-1 | A-1 | A-1 | A-1 |
|  | Content [part by mass] | 24.5 | 24.5 | 25.3 | 25.3 | 23.9 | 23.9 | 29.4 | 29.4 | 29.4 | 29.4 | 29.4 | 29.4 | 24 |
| Curing agent | Type | B-1 | B-1 | B-1 | B-1 | B-1 | B-1 | B-2 | B-2 | B-3 | B-3 | B-4 | B-4 | B-5 |
|  | Content [part by mass] | 5.5 | 5.5 | 4.7 | 4.7 | 6.1 | 6.1 | 0.6 | 0.6 | 0.6 | 0.6 | 0.6 | 0.6 | 6 |
| Particle | Type | Fe | Fe | Fe | Fe | Fe | Fe | Fe | Fe | Fe | Fe | Fe | Fe | Fe |
|  | Content [part by mass] | 70 | 70 | 70 | 70 | 70 | 70 | 70 | 70 | 70 | 70 | 70 | 70 | 70 |
| 100 × b/c [%] | | 33 | 27 | 31 | 27 | 34 | 28 | 32 | 26 | 32 | 26 | 30 | 26 | 33 |
| Applied pressure [MPa] | | 300 | 200 | 300 | 200 | 300 | 200 | 300 | 200 | 300 | 200 | 300 | 200 | 300 |
| Pressurization form | | Cold | Cold | Cold | Cold | Cold | Cold | Cold | Cold | Cold | Cold | Cold | Cold | Cold |
| Acoustic velocity | | C | D | C | D | C | D | C | D | C | D | C | D | B |
| Variations in AI | | A | A | A | A | A | A | A | A | A | A | A | A | A |

[Table 2]

Table 1-3

|  |  | E21 | E22 | E23 | E24 | E25 | E26 | E27 | E28 | E29 |
|---|---|---|---|---|---|---|---|---|---|---|
| Resin | Type | A-1 | A-1 | A-1 | A-2 | A-2 | A-3 | A-3 | A-6 | A-7 |
|  | Conten t [part by mass] | 25.8 | 25.8 | 21 | 29.4 | 29.4 | 24 | 24 | 22.5 | 50 |
| Curing agent | Type | B-6 | B-6 | B-7 | B-8 | B-8 | B-9 | B-9 | B-11 | - |
|  | Conten t [part by mass] | 4.2 | 4.2 | 9 | 0.6 | 0.6 | 6 | 6 | 7.5 | - |

(continued)

| Table 1-3 | | | E21 | E22 | E23 | E24 | E25 | E26 | E27 | E28 | E29 |
|---|---|---|---|---|---|---|---|---|---|---|---|---|
| Particle | Type | | Fe | Fe | Fe | Fe | Fe | Fe | Fe | Fe | Fe |
| | Conten t [part by mass] | | 70 | 70 | 70 | 70 | 70 | 70 | 70 | 70 | 70 |
| 100 × b/c [%] | | | 34 | 28 | 32 | 31 | 26 | 32 | 26 | 33 | 33 |
| Applied pressure [MPa] | | | 300 | 200 | 300 | 300 | 200 | 300 | 200 | 300 | 300 |
| Pressurization form | | | Cold | Cold | Cold | Cold | Cold | Cold | Cold | Cold | Cold |
| Acoustic velocity | | | C | D | B | C | D | C | D | D | D |
| Variations in Al | | | A | A | A | A | A | A | A | A | A |

| Table 1-4 | | | E30 | E31 | E32 | E33 | E34 | E35 | E36 | C7 | C8 |
|---|---|---|---|---|---|---|---|---|---|---|---|
| Resin | Type | | A-1 | A-1 | A-1 | A-1 | A-1 | A-1 | A-1 | A-5 | A-5 |
| | Content [part by mass] | | 23.9 | 6.4 | 6.4 | 4 | 4 | 3.2 | 3.2 | 28.8 | 14.4 |
| Curing agent | Type | | B-1 | B-1 | B-1 | B-1 | B-1 | B-1 | B-1 | B-10 | B-10 |
| | Content [part by mass] | | 6.1 | 1.6 | 1.6 | 1 | 1 | 0.8 | 0.8 | 7.2 | 3.6 |
| Particle | Type | | Fe | Mo | Mo | WC | WC | W | W | Fe | Fe |
| | Content [part by mass] | | 70 | 92 | 92 | 95 | 95 | 96 | 96 | 64 | 82 |
| 100 × b/c [%] | | | 26 | 51 | 45 | 55 | 50 | 50 | 42 | 8 | 15 |
| Applied pressure [MPa] | | | 100 | 100 | 50 | 100 | 50 | 100 | 50 | 9 | 9 |
| Pressurization form | | | Hot | Hot | Hot | Hot | Hot | Hot | Hot | Vacuum electric heating | Vacuum electric heating |
| Acoustic velocity | | | D | A | A | A | A | A | B | F | F |
| Variations in Al | | | A | A | B | A | A | A | A | C | C |

| Table 1-5 | | | E37 | E38 | E39 | E40 | C9 | E41 | E42 | E43 | E44 | C10 | E45 | E46 | E47 | E48 | C11 |
|---|---|---|---|---|---|---|---|---|---|---|---|---|---|---|---|---|---|
| Resin | Type | | A-1 | A-1 | A-1 | A-1 | A-1 | A-1 | A-1 | A-1 | A-1 | A-1 | A-1 | A-1 | A-1 | A-1 | A-1 |
| | Content [part by mass] | | 31.9 | 23.9 | 16 | 8 | 8 | 31.9 | 23.9 | 16 | 8 | 8 | 31.9 | 23.9 | 16 | 8 | 8 |
| Curing agent | Type | | B-1 | B-1 | B-1 | B-1 | B-1 | B-1 | B-1 | B-1 | B-1 | B-1 | B-1 | B-1 | B-1 | B-1 | B-1 |
| | Content [part by mass] | | 8.1 | 6.1 | 4 | 2 | 2 | 8.1 | 6.1 | 4 | 2 | 2 | 8.1 | 6.1 | 4 | 2 | 2 |
| Particle | Type | | Co | Co | Co | Co | Co | Zr | Zr | Zr | Zr | Zr | Ti | Ti | Ti | Ti | Ti |
| | Content [part by mass] | | 60 | 70 | 80 | 90 | 90 | 60 | 70 | 80 | 90 | 90 | 60 | 70 | 80 | 90 | 90 |
| 100 × b/c [%] | | | 25 | 34 | 48 | 69 | 24 | 25 | 32 | 45 | 62 | 24 | 23 | 30 | 42 | 58 | 23 |
| Applied pressure [MPa] | | | 300 | 300 | 300 | 300 | 0 | 300 | 300 | 300 | 300 | 0 | 300 | 300 | 300 | 300 | 0 |
| Pressurization form | | | Cold | Cold | Cold | Cold | - | Cold | Cold | Cold | Cold | - | Cold | Cold | Cold | Cold | - |
| Acoustic velocity | | | D | C | B | A | - | D | C | B | B | - | D | C | B | B | - |
| Variations in Al | | | A | A | A | A | C | A | A | A | A | C | A | A | A | A | C |

EP 4 223 226 B1

| Table 1-6 | | | C12 | E49 | E50 | E51 | E52 | E53 | E54 | E55 | E56 | E57 | C13 |
|---|---|---|---|---|---|---|---|---|---|---|---|---|---|
| Resin | Type | | A-1 | A-1 | A-1 | A-1 | A-1 | A-1 | A-1 | A-1 | A-1 | A-1 | A-1 |
| | Content [part by mass] | | 31.9 | 23.9 | 23.9 | 16 | 6.4 | 6.4 | 6.4 | 6.4 | 6.4 | 6.4 | 6.4 |
| Curing agent | Type | | B-1 | B-1 | B-1 | B-1 | B-1 | B-1 | B-1 | B-1 | B-1 | B-1 | B-1 |
| | Content [part by mass] | | 8.1 | 6.1 | 6.1 | 4 | 1.6 | 1.6 | 1.6 | 1.6 | 1.6 | 1.6 | 1.6 |
| Particle | Type | | Mo | Mo | Mo | Mo | Mo | Mo | Mo | Mo | Mo | Mo | Mo |
| | Content [part by mass] | | 60 | 70 | 70 | 80 | 92 | 92 | 92 | 92 | 92 | 92 | 92 |
| 100 × b/c [%] | | | 20 | 30 | 26 | 35 | 67 | 54 | 46 | 40 | 30 | 25 | 23 |
| Applied pressure [MPa] | | | 300 | 300 | 200 | 300 | 300 | 200 | 100 | 50 | 25 | 10 | 0 |
| Pressurization form | | | Cold | Cold | Cold | Cold | Cold | Cold | Cold | Cold | Cold | Cold | - |
| Acoustic velocity | | | E | C | D | B | A | A | B | B | C | D | - |
| Variations in AI | | | A | A | A | A | A | A | A | B | B | B | C |

[Table 4]

| Table 1-7 | | C14 | E58 | E59 | E60 | E61 | E62 | E63 | E64 | E65 | C15 |
|---|---|---|---|---|---|---|---|---|---|---|---|
| Resin | Type | A-1 | A-1 | A-1 | A-1 | A-1 | A-1 | A-1 | A-1 | A-1 | A-1 |
| | Content [part by mass] | 23.9 | 15.9 | 15.9 | 4 | 4 | 4 | 4 | 4 | 4 | 4 |
| Curing agent | Type | B-1 | B-1 | B-1 | B-1 | B-1 | B-1 | B-1 | B-1 | B-1 | B-1 |
| | Content [part by mass] | 6.1 | 4.1 | 4.1 | 1 | 1 | 1 | 1 | 1 | 1 | 1 |
| Particle | Type | WC | WC | WC | WC | WC | WC | WC | WC | WC | WC |
| | Content [part by mass] | 70 | 80 | 80 | 95 | 95 | 95 | 95 | 95 | 95 | 95 |
| 100 × b/c [%] | | 15 | 29 | 33 | 65 | 51 | 50 | 46 | 35 | 25 | 22 |
| Applied pressure [MPa] | | 300 | 300 | 330 | 300 | 200 | 100 | 50 | 25 | 10 | 0 |
| Pressurization form | | Cold | Cold | Cold | Cold | Cold | Cold | Cold | Cold | Cold | - |
| Acoustic velocity | | E | C | B | A | A | A | B | C | D | - |
| Variations in AI | | A | A | A | A | A | A | A | B | B | B |

| Table 1-8 | | C16 | E66 | E67 | E68 | E69 | E70 | E71 | E72 | C17 |
|---|---|---|---|---|---|---|---|---|---|---|
| Resin | Type | A-1 | A-1 | A-1 | A-1 | A-1 | A-1 | A-1 | A-1 | A-1 |
| | Content [part by mass] | 23.9 | 15.9 | 3.2 | 3.2 | 3.2 | 3.2 | 3.2 | 3.2 | 3.2 |
| Curing agent | Type | B-1 | B-1 | B-1 | B-1 | B-1 | B-1 | B-1 | B-1 | B-1 |
| | Content [part by mass] | 6.1 | 4.1 | 0.8 | 0.8 | 0.8 | 0.8 | 0.8 | 0.8 | 0.8 |

(continued)

| Table 1-8 | | | | | | | | | | |
|---|---|---|---|---|---|---|---|---|---|---|
| | | C16 | E66 | E67 | E68 | E69 | E70 | E71 | E72 | C17 |
| Particle | Type | W | W | W | W | W | W | W | W | W |
| | Content [part by mass] | 70 | 80 | 96 | 96 | 96 | 96 | 96 | 96 | 96 |
| $100 \times b/c$ [%] | | 13 | 27 | 64 | 50 | 40 | 35 | 27 | 25 | 21 |
| Applied pressure [MPa] | | 300 | 300 | 300 | 200 | 100 | 50 | 25 | 10 | 0 |
| Pressurization form | | Cold | Cold | Cold | Cold | Cold | Cold | Cold | Cold | - |
| Acoustic velocity | | E | C | A | A | B | B | C | D | - |
| Variations in AI | | A | A | A | A | A | A | B | B | C |

<Notes of table>

"E": Example
"C": Comparative Example
"Resin": thermosetting resin
"Particle": inorganic filler particles

[Thermosetting resin]

(A-1) Bisphenol A diglycidyl ether ("jER825" (trade name) manufactured by Mitsubishi Chemical Corporation, epoxy equivalent: 170)
(A-2) Bisphenol A diglycidyl ether ("jER828" (trade name) manufactured by Mitsubishi Chemical Corporation, epoxy equivalent: 190)
(A-3) Bisphenol A diglycidyl ether ("jER834" (trade name) manufactured by Mitsubishi Chemical Corporation, epoxy equivalent: 230)
(A-4) Bisphenol F diglycidyl ether ("EPICLON 830" (trade name) manufactured by DIC Corporation, epoxy equivalent: 170)
(A-5) Epoxy resin ("C1001A" (trade name) manufactured by TESK Co., Ltd.)
(A-6) Urethane resin ("TAKENATE A-242B" (trade name) manufactured by Mitsui Chemicals Inc.)
(A-7) Phenol resin ("J-325 (60 wt% methanol solution)" (trade name) manufactured by DIC Corporation)

[Curing agent]

(B-1) Isophorone diamine
(B-2) Triethylene tetramine
(B-3) 2,4,6-Tris(dimethylaminomethyl)phenol (manufactured by Nacalai Tesque Inc., trade name: "LUVEAK DMP-30")
(B-4) Polyamide amine (manufactured by DIC Corporation, trade name: "LUCKAMIDE EA-330")
(B-5) Menthenediamine
(B-6) m-Phenylenediamine
(B-7) Polyetheramine T-403 (trade name, manufactured by BASF SE)
(B-8) 2-Ethyl-4-methylimidazole
(B-9) Hexahydrophthalic anhydride (manufactured by New Japan Chemical Co., Ltd., trade name: "RIKACID HH")
(B-10) C1001B (trade name, manufactured by TESK Co., Ltd.)
(B-11) TAKENATE A-242A (manufactured by Mitsui Chemicals Inc.)

## EP 4 223 226 B1

[Inorganic filler particles]

Fe: EW-I (particle size: 2 $\mu$m, density (25°C): 7.9 g/cm$^3$) trade name, manufactured by BASF SE)
Co: Cobalt powder S-series (particle size: 4 $\mu$m, density (25°C): 8.9 g/cm$^3$)) (trade name, manufactured by Freeport Cobalt)
Zr: Rc-100 zirconium oxide (particle size: 1 to 4 $\mu$m, density (25°C): 6.0 g/cm$^3$)) (trade name, manufactured by DAIICHI KIGENSO KAGAKU KOGYO CO., LTD.)
Mo: Molybdenum powder Mo-6 (particle size: 6 $\mu$m, density (25°C): 10.3 g/cm$^3$)) (trade name, manufactured by JAPAN NEW METALS CO., LTD.)
WC: Uniform-grained tungsten carbide powder (particle size: 9 $\mu$m, density (25°C): 15.8 g/cm$^3$)) (manufactured by A.L.M.T. Corp.)
W: Uniform-grained tungsten powder (particle size: 5 $\mu$m, density (25°C): 19.3 g/cm$^3$)) (manufactured by A.L.M.T. Corp.)
Ti: Large-particle-size titanium oxide (particle size: 1 $\mu$m, density (25°C): 4.2 g/cm$^3$)) (manufactured by FUJI TITANIUM INDUSTRY CO., LTD.)

From the results shown in Table 1, the following is found.

In the acoustic matching sheets of Comparative Examples 2 to 6, 12, 14, and 16 produced through the isotropically pressing process, it does not meet the grain cross-sectional area requirement of the present invention. These acoustic matching sheets were inferior in the proportion of increase in acoustic velocity. In addition, in the acoustic matching sheets of Comparative Examples 7 and 8 produced through the vacuum electric heating pressing process, it does not meet the grain cross-sectional area requirement of the present invention, and the proportion of increase in acoustic velocity was inferior.

On the other hand, it is found that, in the acoustic matching sheets according to the present invention (Examples 1 to 72), the increase in acoustic velocity is large and the variations in acoustic characteristics are small.

The present invention has been described with the embodiments thereof, any details of the description of the present invention are not limited unless described otherwise. It should be noted and understood that improvements and modifications of the embodiments described above may be made, within the scope of the appended claims.

The present application claims the priority of JP2020-166114 filed in Japan on September 30, 2020.

Explanation of References

1: acoustic lens
2: acoustic matching layer
3: piezoelectric element layer
4: backing material
7: housing
9: cord
10: ultrasound probe

**Claims**

1. An acoustic matching layer material comprising:

   a thermosetting resin component; and
   inorganic filler particles,
   **characterised in that**
   in a cross-sectional observation of the acoustic matching layer material, a cross-sectional area of the inorganic filler particles satisfies Expression (1),

$$\text{Expression (1)}\ 100 \times b/c \geq 25$$

b: in a case where a number-average value of cross-sectional areas of the inorganic filler particles is denoted as a, a total cross-sectional area of particles having a cross-sectional area 7 times or more of a in the inorganic filler particles is denoted as b

c: a total cross-sectional area of the inorganic filler particles is denoted as c.

2. The acoustic matching layer material according to claim 1,
wherein a density of the inorganic filler particles at 25°C is 7.5 to 21.5 g/cm$^3$.

3. The acoustic matching layer material according to claim 1 or 2,
wherein the inorganic filler particles include metal particles.

4. The acoustic matching layer material according to claim 3,
wherein metal atoms constituting the metal particles include at least one metal atom of periodic table Groups 4 to 12.

5. The acoustic matching layer material according to any one of claims 1 to 4,
wherein a content of the inorganic filler particles in the acoustic matching layer material is 60% to 96% by mass.

6. The acoustic matching layer material according to any one of claims 1 to 5,
wherein the thermosetting resin component includes an epoxy resin component.

7. The acoustic matching layer material according to any one of claims 1 to 6, wherein Expression (1) is Expression (3) or Expression (4):

$$\text{Expression (3)} \; 80 \geq 100 \times b/c \geq 30$$

$$\text{Expression (4)} \; 80 \geq 100 \times b/c \geq 50$$

8. An acoustic matching sheet consisting of the acoustic matching layer material according to any one of claims 1 to 7.

9. A composition for forming an acoustic matching sheet, which is used for forming the acoustic matching sheet according to claim 8, the composition comprising:

   a thermosetting resin;
   a curing agent; and
   an inorganic filler.

10. An acoustic wave probe comprising:
   the acoustic matching sheet according to claim 8 as an acoustic matching layer.

11. An acoustic wave measurement apparatus comprising:
   the acoustic wave probe according to claim 10.

12. The acoustic wave measurement apparatus according to claim 11,
   wherein the acoustic wave measurement apparatus is an ultrasound diagnostic apparatus.

13. A manufacturing method of an acoustic matching layer material including a thermosetting resin component and inorganic filler particles, the manufacturing method comprising:

   isotropically pressing a composition for forming an acoustic matching layer, which includes a thermosetting resin, a curing agent, and an inorganic filler,
   **characterised in that**
   in a cross-sectional observation of the acoustic matching layer material, a cross-sectional area of the inorganic filler particles satisfies Expression (1),

$$\text{Expression (1)} \; 100 \times b/c \geq 25$$

b: in a case where a number-average value of cross-sectional areas of the inorganic filler particles is denoted as a, a total cross-sectional area of particles having a cross-sectional area 7 times or more of a in the inorganic filler particles is denoted as b

c: a total cross-sectional area of the inorganic filler particles is denoted as c.

14. A manufacturing method of an acoustic wave probe, comprising:
forming an acoustic matching layer using the acoustic matching layer material according to any one of claims 1 to 7.

**Patentansprüche**

1. Akustisches Anpassungsschichtmaterial, umfassend:

   eine duroplastische Harzkomponente; und
   anorganische Füllstoffpartikel,
   **dadurch gekennzeichnet, dass**
   bei einer Querschnittsbeobachtung des akustischen Anpassungsschichtmaterial eine Querschnittsfläche der anorganischen Füllstoffpartikel Ausdruck (1) erfüllt,

$$\text{Ausdruck (1)} \quad 100 \times b/c \geq 25$$

   b: in einem Fall, in dem ein zahlenmittlerer Wert von Querschnittsflächen der anorganischen Füllstoffpartikel als a bezeichnet wird, eine Gesamtquerschnittsfläche von Partikeln mit einer Querschnittsfläche, die 7-mal oder mehr als a beträgt, bei den anorganischen Füllstoffpartikeln als b bezeichnet wird
   c: eine Gesamtquerschnittsfläche der anorganischen Füllstoffpartikel als c bezeichnet wird.

2. Akustisches Anpassungsschichtmaterial nach Anspruch 1,
   wobei eine Dichte der anorganischen Füllstoffpartikel bei 25 °C 7,5 bis 21,5 g/cm$^3$ beträgt.

3. Akustisches Anpassungsschichtmaterial nach Anspruch 1 oder 2,
   wobei die anorganischen Füllstoffpartikel Metallteilchen enthalten.

4. Akustisches Anpassungsschichtmaterial nach Anspruch 3,
   wobei Metallatome, die die Metallteilchen bilden, mindestens ein Metallatom von Periodensystemgruppen 4 bis 12 enthalten.

5. Akustisches Anpassungsschichtmaterial nach einem der Ansprüche 1 bis 4,
   wobei ein Gehalt der anorganischen Füllstoffpartikel in dem akustischen Anpassungsschichtmaterial 60 Massen-% bis 96 Massen-% beträgt.

6. Akustisches Anpassungsschichtmaterial nach einem der Ansprüche 1 bis 5,
   wobei die duroplastische Harzkomponente eine Komponente von Epoxidharz enthält.

7. Akustisches Anpassungsschichtmaterial nach einem der Ansprüche 1 bis 6, wobei Ausdruck (1) Ausdruck (3) oder Ausdruck (4) ist:

$$\text{Ausdruck (3)} \quad 80 \geq 100 \times b/c \geq 30$$

$$\text{Ausdruck (4)} \quad 80 \geq 100 \times b/c \geq 50.$$

8. Akustisches Anpassungsblatt, das aus dem akustischen Anpassungsschichtmaterial nach einem der Ansprüche 1 bis 7 besteht.

9. Zusammensetzung zum Bilden eines akustischen Anpassungsblatts, das zum Bilden des akustischen Anpassungsblatts nach Anspruch 8 verwendet wird, wobei die Zusammensetzung umfasst:

   ein duroplastisches Harz;

ein Aushärtungsmittel; und
einen anorganischen Füllstoff.

10. Akustische Wellensonde, umfassend:
das akustische Anpassungsblatt nach Anspruch 8 als eine akustische Anpassungsschicht.

11. Akustische Wellenmessvorrichtung, umfassend:
die akustische Wellensonde nach Anspruch 10.

12. Akustische Wellenmessvorrichtung nach Anspruch 11,
wobei die akustische Wellenmessvorrichtung eine Ultraschalldiagnosevorrichtung ist.

13. Herstellungsverfahren eines akustischen Anpassungsschichtmaterials, das duroplastische Harzkomponentez und anorganische Füllstoffpartikel enthält, wobei das Herstellungsverfahren umfasst:

isotropes Pressen einer Zusammensetzung zum Bilden einer akustischen Anpassungsschicht, die ein duroplastisches Harz, ein Härtungsmittel und einen anorganischen Füllstoff enthält,
**dadurch gekennzeichnet, dass**
bei einer Querschnittsbeobachtung des akustischen Anpassungsschichtmaterial eine Querschnittsfläche der anorganischen Füllstoffpartikel Ausdruck (1) erfüllt,

$$\text{Ausdruck (1)} \quad 100 \times b/c \geq 25$$

b: in einem Fall, in dem ein zahlenmittlerer Wert von Querschnittsflächen der anorganischen Füllstoffpartikel als a bezeichnet wird, eine Gesamtquerschnittsfläche von Partikeln mit einer Querschnittsfläche, die 7-mal oder mehr als a beträgt, bei den anorganischen Füllstoffpartikeln als b bezeichnet wird
c: eine Gesamtquerschnittsfläche der anorganischen Füllstoffpartikel als c bezeichnet wird.

14. Herstellungsverfahren einer akustischen Wellensonde, umfassend:
Bilden einer akustischen Anpassungsschicht unter Verwendung des akustischen Anpassungsschichtmaterials nach einem der Ansprüche 1 bis 7.

**Revendications**

1. Matériau de couche d'adaptation acoustique comprenant :

une composante de résine thermodurcissable ; et
des particules de charge inorganiques,
**caractérisé en ce que**
dans une observation de section transversale du matériau de couche d'adaptation acoustique, une surface de section transversale des particules de charge inorganiques satisfait Expression (1),

$$\text{Expression (1)} \quad 100 \times b/c \geq 25$$

b : dans un cas où une valeur moyenne en nombre de surfaces de section transversale des particules de charge inorganiques est désignée par a, une surface de section transversale totale des particules ayant une surface de section transversale supérieure ou égale à 7 fois a dans les particules de charge inorganiques est désignée par b
c : une surface de section transversale totale des particules de charge inorganiques est désignée par c.

2. Matériau de couche d'adaptation acoustique selon la revendication 1,
dans lequel une densité des particules de charge inorganiques à 25 °C est de 7,5 à 21,5 g/cm$^3$.

3. Matériau de couche d'adaptation acoustique selon la revendication 1 ou la revendication 2,
dans lequel les particules de charge inorganiques incluent des particules métalliques.

4. Matériau de couche d'adaptation acoustique selon la revendication 3,

dans lequel des atomes métalliques constituant les particules métalliques incluent au moins un atome métallique des groupes 4 à 12 du tableau périodique.

5. Matériau de couche d'adaptation acoustique selon l'une quelconque des revendications 1 à 4, dans lequel une teneur en particules de charge inorganiques dans le matériau de couche d'adaptation acoustique est de 60 % à 96 % en masse.

6. Matériau de couche d'adaptation acoustique selon l'une quelconque des revendications 1 à 5, dans lequel la composante de résine thermodurcissable inclut une composante de résine époxy.

7. Matériau de couche d'adaptation acoustique selon l'une quelconque des revendications 1 à 6, dans lequel Expression (1) est Expression (3) ou Expression (4) :

$$\text{Expression (3) } 80 \geq 100 \times b/c \geq 30$$

$$\text{Expression (4) } 80 \geq 100 \times b/c \geq 50.$$

8. Feuille d'adaptation acoustique constituée du matériau de couche d'adaptation acoustique selon l'une quelconque des revendications 1 à 7.

9. Composition pour former une feuille d'adaptation acoustique, qui est utilisée pour former la feuille d'adaptation acoustique selon la revendication 8, la composition comprenant :

   une résine thermodurcissable ;
   un agent de durcissement ; et
   une charge inorganique.

10. Sonde à ondes acoustiques comprenant :
    la feuille d'adaptation acoustique selon la revendication 8 en tant que couche d'adaptation acoustique.

11. Appareil de mesure d'ondes acoustiques comprenant :
    la sonde à ondes acoustiques selon la revendication 10.

12. Appareil de mesure d'ondes acoustiques selon la revendication 11,
    dans lequel l'appareil de mesure d'ondes acoustiques est un appareil de diagnostic par ultrasons.

13. Procédé de fabrication d'un matériau de couche d'adaptation acoustique incluant un composant de résine thermodurcissable et des particules de charge inorganiques, le procédé de fabrication comprenant :

    presser de manière isotrope une composition pour former une couche d'adaptation acoustique, qui inclut une résine thermodurcissable, un agent de durcissement et une charge inorganique,
    **caractérisé en ce que**
    dans une observation de section transversale du matériau de couche d'adaptation acoustique, une surface de section transversale des particules de charge inorganiques satisfait Expression (1),

$$\text{Expression (1) } 100 \times b/c \geq 25$$

    b : dans un cas où une valeur moyenne en nombre de surfaces de section transversale des particules de charge inorganiques est désignée par a, une surface de section transversale totale des particules ayant une surface de section transversale supérieure ou égale à 7 fois a dans les particules de charge inorganiques est désignée par b
    c : une surface de section transversale totale des particules de charge inorganiques est désignée par c.

14. Procédé de fabrication d'une sonde à ondes acoustiques, comprenant :
    former une couche d'adaptation acoustique en utilisant le matériau de couche d'adaptation acoustique selon l'une quelconque des revendications 1 à 7.

# FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019088145 A
- JP 2014168489 A
- US 2007205698 A
- US 2015173625 A

- WO 2019088148 A
- JP 2011071842 A
- JP 2020166114 A